# EUROPEAN PATENT SPECIFICATION

(11) **EP 0 748 791 B1**
(45) Date of publication and mention of the grant of the patent: **17.11.1999**
(21) Application number: 96304313.8
(22) Date of filing: 07.06.1996
(51) Int. Cl.: C07C 227/40, C07C 227/42, C07C 229/08

(54) **Valine p-isopropylbenzene sulfonate and a process for purifying valine**
Kristallines Valin-p-Isopropylbenzolsulfonat und ein Verfahren zur Reinigung von Valin
Cristal de valine-p-isopropylbenzène sulfonate et un procédé de purification de la valine

(30) Priority: 12.06.1995 JP 14484395
(43) Date of publication of application: 18.12.1996
(73) Proprietor: Ajinomoto Co., Inc., Tokyo 104 (JP)
(72) Inventor: Hasegawa, Kazuhiro, Ajinomoto Co., Inc., Kawasaki-ku, Kawasaki-shi, Kanagawa-ken (JP); Kaneko, Tetsuya, Ajinomoto Co., Inc., Kawasaki-ku, Kawasaki-shi, Kanagawa-ken (JP); Takahashi, Noriko, Ajinomoto Co., Inc., Kawasaki-ku, Kawasaki-shi, Kanagawa-ken (JP); Sano, Chiaki, Ajinomoto Co., Inc., Kawasaki-ku, Kawasaki-shi, Kanagawa-ken (JP)
(74) Representative: Nicholls, Kathryn Margaret

(56) References cited:
- PATENT ABSTRACTS OF JAPAN vol. 011, no. 307 (C-450), 7 October 1987 & JP 62 096454 A (HIROYUKI NOHIRA), 2 May 1987,
- PATENT ABSTRACTS OF JAPAN vol. 001, no. 045 (C-011), 4 May 1977 & JP 52 003016 A (TANABE SEIYAKU CO LTD), 11 January 1977,

## Description

The present invention relates to a novel valine isopropylbenzenesulfonate crystal which is suitable for the purification of valine and a process for purifying valine using the valine isopropylbenzenesulfonate. L-valine is useful as a starting material for medical amino acid preparations and as a synthetic intermediate of various medications. Further, D-valine is useful as an intermediate of agricultural chemicals.

Valine is produced by a method in which proteins such as soybean protein and the like are hydrolyzed or a method in which microorganisms having ability to produce valine are incubated. The conventional method of separating valine from the valine-containing aqueous solutions such as protein hydrolyzates, fermentation solutions and the like obtained by the above-mentioned methods and purifying the same includes the following:
(1) method in which after acid or basic amino acids are separated and removed through treatment using an ion-exchange resin, a neutral amino acid fraction is obtained and repeatedly recrystallized to remove the neutral amino acids other than valine [Biochem. J., 48, 313 (1951)].
(2) method in which hydrochloric acid is added to a valine-containing aqueous solution to form and precipitate valine hydrochloride crystals, and this procedure is repeated [Japanese Laid-Open Patent Application (Kokai) No. 16,450/1981].

However, these methods have involved problems. That is, the former method is quite intricate and can hardly separate valine from leucine and isoleucine. In the latter method, the solubility of valine hydrochloride crystals in water is so high that the yield of valine is decreased.

In another purification method, valine is purified by reacting it with a precipitant such as tetrachloroorthophthalic acid, sulfoisophthalic acid, flavianic acid or the like which selectively forms an adduct with valine (Japanese Patent Publication No. 25,059/1967). However, this method has suffered the problems that the precipitant used is expensive and industrially hard to obtain, that since the solubility of the resulting adduct is high, it is difficult to recover valine in high yield, and that isolation of valine from the adduct is complicated.

Further, as a precipitant of leucine which is a neutral amino acid having the structure similar to valine, 1,2-dimethylbenzenesulfonic acid (Japanese Patent Publication No. 11,373/1965) and p-toluenesulfonic acid [Japanese Laid-Open Patent Application (Kokai) No. 3,016/1977] have been known to date. However, since these precipitants are specific to leucine, they can hardly be used as precipitants of valine. Thus, the precipitants are specific substances, and it is not easy to find a precipitant specific to valine.

In JP-A-62096454 a process is described wherein DL-valine is reacted with p-xylenesulfonate to achieve optical resolution of the valine.

The present invention addresses the need to provide a simple process for purifying valine having a high purity in high yield using an inexpensive precipitant.

Under these circumstances, the present inventors have conducted studies, and have consequently found that when a valine-containing aqueous solution is reacted with p-isopropylbenzenesulfonic acid and then cooled, valine p-isopropylbenzenesulfonate crystals which are sparingly-soluble salts are precipitated selectively. This finding has led to the completion of the present invention.

That is, the present invention relates to:
(1) a valine p-isopropylbenzenesulfonate crystal comprising 1 mol of valine and 1 mol of p-isopropylbenzenesulfonic acid, and
(2) a process for purifying valine, which comprises reacting a valine-containing aqueous solution with p-isopropylbenzenesulfonic acid or its water-soluble salt to form a valine p-isopropylbenzenesulfonate crystal, then separating the valine p-isopropylbenzenesulfonate, and dissociating the valine p-isopropylbenzenesulfonate to obtain valine.

Valine which can be used in the process of the present invention may be an optically active substance which is an L-isomer or a D-isomer, a racemic modification or a mixture thereof. Examples of the valine-containing aqueous solution include an amino acid mixed solution obtained by separating and removing basic amino acids from a hydrolyzate formed by hydrolyzing proteins such as soybean protein and the like, a fermentation solution obtained by incubating microorganisms having ability to form and accumulate valine, a solution obtained by removing cells from this fermentation solution, a solution obtained by treating the above-mentioned solution through an ion-exchange resin or an adsorption resin, and an aqueous solution of crude DL-valine obtained by a synthesis method in which hydantoin derivatives are formed during the reaction.

p-Isopropylbenzenesulfonic acid which is used in the present invention can be easily formed by charging isopropylbenzene and conc. sulfuric acid in an amount of 1.5 mols per mol of isopropylbenzene into a glass container, and heating the mixture at 120°C for from 2 to 3 hours, and it can be easily obtained industrially at low cost. Further, p-isopropylbenzenesulfonic acid may be used in the form of a free acid, or as its water-soluble salt, for example, an alkali metal salt such as a sodium salt, a potassium salt or the like or an alkaline-earth metal salt such as a calcium salt or the like, or as an ammonium salt. p-Isopropylbenzenesulfonic acid or its water-soluble salt is preferably used in an amount which is equimolar to or more than the amount of valine contained in the aqueous solution, preferably from 1.0 to 1.1 mols per mol of valine. There is no need to use valine in large excess.

The valine p-isopropylbenzenesulfonate crystal which is a desired sparingly-soluble addition compound can be formed and precipitated by adding p-isopropylbenzenesulfonic acid or its water-soluble salt to an aqueous solution containing 3 g/dl or more of valine and adjusting the pH to approximately 1.5. The pH of the solution which is suitable for formation and precipitation of the valine p-isopropylbenzenesulfonate crystal is between 0.1 and 2.3, especially preferably between 1.0 and 1.7. The acid which is used to adjust the pH includes inorganic acids such as hydrochloric acid and sulfuric acid. When a seed crystal of valine p-isopropylbenzenesulfonate is added to a mixed solution of p-isopropylbenzenesulfonic acid as required, the crystal can be precipitated efficiently. When the valine aqueous solution is dilute, the valine p-isopropylbenzenesulfonate crystal can be precipitated through concentration. In this case, p-isopropylbenzenesulfonic acid may be added at any stage before or after the concentration. When the valine aqueous solution is concentrated under neutral conditions, the crystal of the free valine is precipitated. The valine isopropylbenzenesulfonate crystal can be easily formed by adding thereto an appropriate amount of p-isopropylbenzenesulfonic acid as such and adjusting the pH to approximately 1.5. Further, the valine p-isopropylbenzenesulfonate crystal can be also precipitated through the concentration by adjusting a pH of a valine dilute solution containing an appropriate amount of p-isopropylbenzenesulfonic acid to approximately 2 in advance, and then concentrating the solution.

The valine p-isopropylbenzenesulfonate crystal precipitated may be separated by usual solid-liquid separation such as filtration, centrifugation or the like. Although the crystal separated has a high purity, it can be further purified by a usual purification method such as washing, dehydration, recrystallization or the like.

The thus-obtained valine p-isopropylbenzenesulfonate crystal comprising 1 mol of valine and 1 mol of p-isopropylbenzenesulfonic acid is a novel product which exhibits the following properties.
White plate crystal. Soluble in water and ethanol.
Solubility in water: 7.8% by weight (pH 1.5, 10°C)
Crystal structure: monoclinic system
Crystal density: 1.28/cm³

| Elemental analysis: | | | | |
|---|---|---|---|---|
| found | C 52.9%, | H 7.3%, | N 4.4%, | S 9.9% |
| calculated | C 53.0%, | H 7.3%, | N 4.4%, | S 10.1% |

In order to isolate the free valine from the valine p-isopropylbenzenesulfonate crystal, the solution containing a large amount of hot water is preferably brought into contact with a weakly basic ion-exchange resin (OH-type), or an alkali such as sodium hydroxide or the like is added thereto. When using the ion-exchange resin, p-isopropylbenzenesulfonic acid is adsorbed thereto and the free valine is obtained in the effluent. This solution may be treated in a usual manner, for example, subjected to crystallization through concentration to obtain the valine crystal. The precipitant (p-isopropylbenzenesulfonic acid) adsorbed on the resin is eluted as an alkali salt when the resin is regenerated with an alkaline solution such as a sodium hydroxide solution or the like.

When using addition of an alkali, the alkali such as sodium hydroxide or the like is added to an aqueous suspension of the valine p-isopropylbenzenesulfonate crystal either as such or in the form of an aqueous solution to adjust the pH to between 5 and 8, preferably between 6 and 7, whereby p-isopropylbenzenesulfonic acid is dissolved in the solution as an alkali salt, the free valine is precipitated, and the valine precipitated is obtained through separation.

The precipitant (p-isopropylbenzenesulfonic acid) which has been separated and recovered as the alkali salt can be reused, as such, as a precipitant in the subsequent reaction.

Embodiments of the present invention will be illustrated specifically by referring to the following Examples. The amounts of valine and other amino acids in the following Examples were determined using a Hitachi L-8500 Model amino acid analyzer. In the accompanying figures:

Fig. 1 is a ¹H-NMR spectrum of sodium p-isopropylbenzenesulfonate obtained in Reference Example 1.

Fig. 2 is a powder X-ray diffraction pattern of L-valine p-isopropylbenzenesulfonate obtained in Example 1.

### Examples

### Reference Example 1

### (Production of p-isopropylbenzenesulfonic acid)

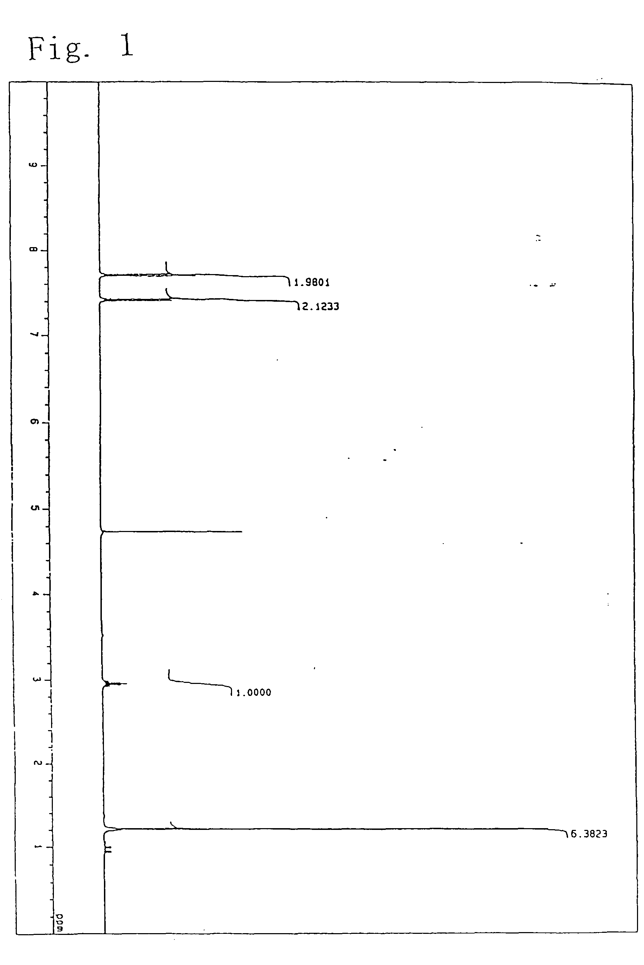

Conc. sulfuric acid (84 ml, 1.5 mols) was added to 140 ml (1 mol) of isopropylbenzene, and the mixture was heat-stirred at a temperature of from 120 to 130°C for 2.5 hours. If unreacted isopropylbenzene remains, layer separation is observed. Unless layer separation was observed, the reaction was completed, and a solution containing p-isopropylbenzenesulfonic acid as a main ingredient was obtained. This solution was charged into 350 ml of water, and was partially neutralized with sodium hydrogencarbonate. Then, p-isopropylbenzenesulfonic acid was converted into a sodium salt with sodium chloride to precipitate a sodium p-isopropylbenzenesulfonate crystal. The thus-obtained crystal was separated through filtration, and then dried under reduced pressure. This sodium p-isopropylbenzenesulfonate was easily soluble in water and slightly soluble in ethanol. The ¹H-NMR spectrum of the thus-obtained crystal is shown in Fig. 1.

### Example 1

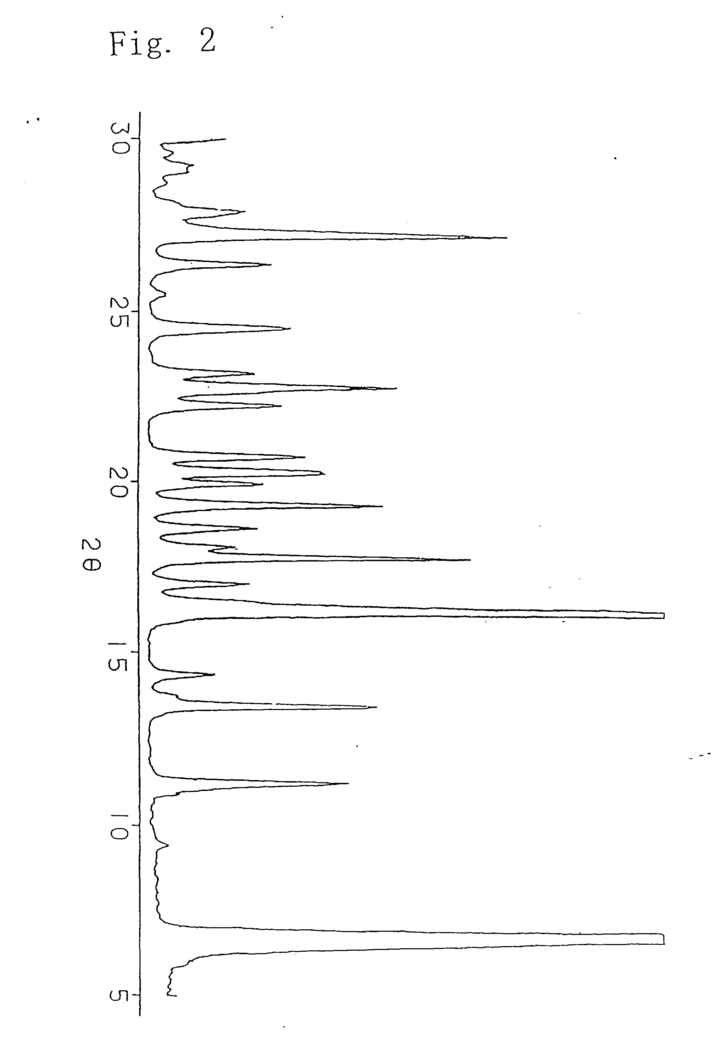

Seventy millilitres of water were added to 10 g of L-valine and 19 g of sodium p-isopropylbenzenesulfonate obtained in Reference Example 1, and the pH of the solution was adjusted to 1.5 with sulfuric acid. Then, the temperature of the solution was raised to 50°C to dissolve the solid therein. Subsequently, the solution was cooled to 10°C to precipitate an L-valine p-isopropylbenzenesulfonate crystal. The crystal precipitated was separated through filtration, and then dried under reduced pressure. The thus-obtained L-valine p-isopropylbenzenesulfonate was a white fine crystal. The crystal system thereof was a monoclinic system, and the specific gravity of the crystal was 1.28 g/cm³. The powder X-ray diffraction pattern of the crystal is shown in Fig. 2. The X-ray diffraction was conducted using Cu-Ka rays as a radiation source. The elemental analysis of the crystal revealed the following composition.
C 53.1%, H 7.2%, N 4.4%, S 10.0%

### Example 2

Sodium p-isopropylbenzenesulfonate (26.6 g, equimolar amount relative to valine) was added to 100 ml of a solution containing 14 g of L-valine, 1.1 g of L-leucine, 1.1 g of L-isoleucine, 1.1 g of L-glutamic acid and 1.1 g of L-alanine, and the pH of the solution was adjusted to 1.5 with sulfuric acid. Then, the mixture was heat-dissolved. Subsequently, the solution was cooled to precipitate an L-valine p-isopropylbenzenesulfonate crystal. The crystal precipitated was recovered by centrifugation, and was then dissolved in a large amount of hot water. The solution was passed through an OH-type weakly basic ion-exchange resin to remove p-isopropylbenzenesulfonic acid. The effluent was crystallized through concentration to obtain 9 g of a free L-valine crystal. The analysis of the mother liquor revealed that the precipitation rate of valine was 70% and the purity of the free L-valine was 97%. The contents of the other amino acids were 3% or less.

### Example 3

Sodium p-isopropylbenzenesulfonate (26.6 g, equimolar amount relative to valine) was added to 100 ml of a solution containing 14 g of L-valine, 0.42 g of L-leucine, 0.42 g of L-isoleucine, 0.42 g of L-glutamic acid and 0.42 g of L-alanine, and the pH of the solution was adjusted to 1.5 with sulfuric acid. Then, the mixture was heat-dissolved. Subsequently, the solution was cooled to precipitate an L-valine p-isopropylbenzenesulfonate crystal. The crystal precipitated was recovered by centrifugation, and was then dissolved in a large amount of hot water. The solution was passed through an OH-type weakly basic ion-exchange resin to remove p-isopropylbenzenesulfonic acid. The effluent was crystallized through concentration to obtain 9 g of a free L-valine crystal. The analysis of the mother liquor revealed that the precipitation rate of valine was 70% and the purity of the free L-valine was 99%. The contents of the other amino acids were 1% or less.

### Example 4

Sodium p-isopropylbenzenesulfonate (19 g, equimolar amount relative to valine) was added to 100 ml of a solution containing 10 g of D-valine, 0.3 g of D-leucine and 0.3 g of D-isoleucine and the pH of the solution was adjusted to 1.5 with sulfuric acid. Then, the mixture was heat-dissolved. Subsequently, the solution was cooled to precipitate 18 g of a D-valine p-isopropylbenzenesulfonate crystal. The crystal precipitated was recovered by centrifugation, and was then dissolved in a large amount of hot water. The solution was passed through an OH-type weakly basic ion-exchange resin to remove p-isopropylbenzenesulfonic acid. The effluent was crystallized through concentration to obtain 6.5 g of a free D-valine crystal. The analysis of the mother liquor revealed that the precipitation rate of valine was 70% and the purity of the free D-valine was 99%. The contents of the other amino acids were 1% or less.

### Example 5

Sodium p-isopropylbenzenesulfonate (19 g, equimolar amount relative to valine) was added to 100 ml of a solution containing 10 g of DL-valine, 0.3 g of L-leucine and 0.3 g of L-isoleucine and the pH of the solution was adjusted to 1.5 with sulfuric acid. Then, the mixture was heat-dissolved. Subsequently, the solution was cooled to precipitate 9 g of a DL-valine p-isopropylbenzenesulfonate crystal. The crystal precipitated was recovered by centrifugation, and was then dissolved in a large amount of hot water. The solution was passed through an OH-type weakly basic ion-exchange resin to remove p-isopropylbenzenesulfonic acid. The effluent was crystallized through concentration to obtain 3 g of a free DL-valine crystal. The analysis of the mother liquor revealed that the precipitation rate of valine was 30% and the purity of the free DL-valine was not less than 99%.

### Comparative Example 1

To 20 g of L-valine were added 32.5 g of p-toluenesulfonic acid monohydrate and 47.5 g of water. The mixture was dissolved at room temperature, and was then cooled to 10°C. A valine p-toluenesulfonate crystal was precipitated in a small amount by this procedure. The solubility of L-valine p-toluenesulfonate in water at 10°C (pH 1.5) was 48% by weight.

### Comparative Example 2

To 15 g of L-valine were added 28.5 g of sodium p-n-propylbenzenesulfonate and 56.5 g of water. After the pH was adjusted to 1.5 with sulfuric acid, the mixture was dissolved at 50°C. Then, the solution was cooled to 10°C to obtain 13 g of L-valine p-n-propylbenzenesulfonate. The analysis of the mother liquor revealed that the precipitation rate of valine was only 30%. The solubility of L-valine p-n-propylbenzenesulfonate in water at 10KC (pH 1.5) was 29% by weight. The thus-obtained crystal was a white fine crystal, the crystal system is a monoclinic system, and the specific gravity of the crystal was 1.27 g/cm³.

### Comparative Example 3

Sodium sulfoisophthalate (34.5 g, equimolar amount relative to L-valine) was added to 150 ml of a solution containing 15 g of L-valine, 1.2 g of L-leucine, 1.2 g of L-isoleucine and 1.2 g of L-glutamic acid. After the pH was adjusted to 1.5, the mixture was heat-dissolved. Then, the solution was cooled to precipitate and separate L-valine sulfoisophthalate. The purity of valine in the L-valine sulfoisophthalate crystal was 98%, and the contents of other amino acids were 2%. The yield of valine obtained was 70%. This was dissolved in a large amount of hot water, and the solution was passed through an OH-type weakly basic ion-exchange resin in order to remove sulfoisophthalic acid and obtain free valine. However, the removal of sulfoisophthalic acid was not fully conducted, and it was difficult to obtain completely free valine by this method. Therefore, the removal of sulfoisophthalic acid was conducted using a strongly acidic ion-exchange resin. However, in this method, the valine crystal was precipitated in the eluate and within the resin column when eluting valine from the strongly acidic ion-exchange resin. It was difficult to remove sulfoisophthalic acid at good efficiency.

As mentioned above, when the valine p-isopropylbenzenesulfonate crystal obtained in the present invention is used to purify valine, valine having a high purity can be produced at low cost by a simple process, and this is quite useful. That is, the procedure of producing the valine p-isopropylbenzenesulfonate crystal and purifying valine has the effects that since the solubility of the valine p-isopropylbenzenesulfonate is low, valine can be obtained at high efficiency, and that valine can be separated at a high rate from amino acids such as leucine and isoleucine, which are usually hard to separate from valine, because of specificity of valine p-isopropylbenzenesulfonate. isopropylbenzenesulfonate. Further, acid can be easily produced industrially by sulfonating inexpensive isopropylbenzene. Accordingly, the present invention can be easily applied industrially at low cost. It is also easy to separate and obtain valine from valine p-isopropylbenzenesulfonate and to recover p-isopropylbenzenesulfonic acid for reuse.

## Claims

1. A valine p-isopropylbenzenesulfonate crystal comprising 1 mol of valine and 1 mol of p-isopropylbenzenesulfonic acid.

2. A process for the preparation of valine p-isopropylbenzene sulfonate crystals comprising reacting a valine-containing aqueous solution with p-isopropylbenzenesulfonic acid or its water-soluble salt.

3. A process for purifying valine, which comprises reacting a valine-containing aqueous solution with p-isopropylbenzenesulfonic acid or its water-soluble salt to form valine p-isopropylbenzenesulfonate crystals, then separating the valine p-isopropylbenzenesulfonate, and dissociating the valine p-isopropylbenzenesulfonate to obtain valine.

4. The process of claim 2 or claim 3, wherein the water-soluble salt of p-isopropylbenzenesulfonic acid is an alkali metal salt.

## Patentansprüche

1. Valin-p-isopropylbenzolsulfonat-Kristall, der 1 Mol Valin und 1 Mol p-Isopropylbenzolsulfonsäure enthält.

2. Verfahren zur Herstellung von Valin-p-isopropylbenzolsulfonat-Kristallen, welches die Umsetzung einer Valin enthaltenden wäßrigen Lösung mit p-Isopropylbenzolsulfonsäure oder einem wasserlöslichen Salz dieser umfaßt.

3. Verfahren zur Reinigung von Valin, welches die Umsetzung einer Valin enthaltenden wäßrigen Lösung mit p-Isopropylbenzolsulfonsäure oder einem wasserlöslichen Salz dieser unter Bildung von Valin-p-isopropylbenzolsulfonat-Kristallen, anschließendes Abtrennen des Valin-p-isopropylbenzolsulfonats und die Spaltung des Valin-p-isopropylbenzolsulfonats zur Bildung von Valin umfaßt.

4. Verfahren nach Anspruch 2 oder Anspruch 3, wobei das wasserlösliche Salz von p-Isopropylbenzolsulfonsäure ein Alkalimetallsalz ist.

## Revendications

1. Cristal de p-isopropylbenzène sulfonate de valine comprenant 1 mole de valine et 1 mmole d'acide p-isopropylbenzène sulfonique.

2. Procédé de préparation de cristaux de p-isopropylbenzène sulfonate de valine consistant à faire réagir une solution aqueuse contenant de la valine avec de l'acide p-isopropylbenzène sulfonique ou son sel hydrosoluble.

3. Procédé de purification de valine consistant à faire réagir une solution aqueuse contenant de la valine avec de l'acide p-isopropylbenzène sulfonique ou son sel hydrosoluble afin de former des cristaux de p-isopropylbenzène sulfonate de valine, puis à séparer le p-isopropylbenzène sulfonate de valine et à dissocier le p-isopropylbenzène sulfonate de valine afin d'obtenir de la valine.

4. Procédé selon la revendication 2 ou la revendication 3, dans lequel le sel hydrosoluble de l'acide p-isopropylbenzène sulfonique est un sel de métal alcalin.
